# FASCICULE DE BREVET EUROPEEN

(11) **EP 2 651 425 B1**
(45) Date de publication et mention de la délivrance du brevet: **17.08.2016**
(21) Numéro de dépôt: 11811054.3
(22) Date de dépôt: 08.12.2011
(51) Int. Cl.: A61K 33/08, A61K 8/26, A61K 8/60, A61K 8/97, A61K 36/03, A61Q 11/00

(54) **COMPOSITION À BASE D'EXTRAIT D'ALGUES ET D'AU MOINS UNE ZÉOLITHE CHARGÉE À L'ARGENT À USAGE BUCCO-DENTAIRE**
ZUSAMMENSETZUNG AUS ALGENEXTRAKTE UND EIN SILBER-ENTHALTENDE ZEOLITH UND BENUTZUNG IN DER MUNDPFLEGE
COMPOSITION COMPRISING EXTRACT OF ALGAE AND AT LEAST ONE SILVER-LOADED ZEOLITE AND USE THEREOF IN ORAL CARE

(30) Priorité: 14.12.2010 FR 1004856
(43) Date de publication de la demande: 23.10.2013
(73) Titulaire: YS Lab, 29000 Quimper (FR)
(72) Inventeur: FAGON, Roxane, F-29140 Saint Yvi (FR); DUTOT, Mélody, F-75020 Paris (FR); HEMON, Marc, F-22490 Plouer Sur Rance (FR)
(74) Mandataire: Ipside
(86) Numéro de dépôt international: PCT/FR2011/052906
(87) Numéro de publication internationale: WO 2012/080622

(56) Documents cités:
- EP-A1- 1 433 388
- EP-A1- 1 977 756
- WO-A2-2006/027248
- DE-A1- 3 416 332
- DE-A1-102007 030 406
- US-A1- 2004 022 806
- US-B1- 7 556 654
- DATABASE WPI Week 200643 Thomson Scientific, London, GB; AN 2006-421398 XP002638081, "extract for treatment of periodontal diseases comprises extract obtained from Ascophyllum nodosum and Fucus vesiculosus algae", & RU 2 275 206 C2 (UNIV N MED) 27 avril 2006 (2006-04-27)

## Description

La présente invention concerne un produit à usage bucco-dentaire.

Plus particulièrement le produit selon la présente invention agit contre la formation de la plaque dentaire.

Dans ce qui suit la composition à base d'extrait d'algues et d'au moins une zéolithe chargée à l'argent à usage bucco-dentaire va être décrite en relation avec la prophylaxie de la plaque dentaire.

La plaque dentaire est une substance extrêmement collante qui se dépose sur les dents. Elle se forme au cours de la journée et pendant la nuit, entre les séances de brossage. Elle est constituée de protéines de la salive, de sucres alimentaires, de très nombreuses bactéries et de leurs toxines. Les bactéries présentes métabolisent les sucres et produisent des acides corrosifs. Lorsqu'elle n'est pas éliminée par le brossage des dents, la plaque dentaire durcit et se calcifie pour constituer le tartre. Seul un détartrage effectué par un dentiste peut l'éliminer.

L'accumulation de plaque dentaire induit un environnement défavorable pour la gencive. Les bactéries constituant la plaque vont exercer des effets négatifs sur les cellules de gencives : stress oxydatif, stress inflammatoire. La production de radicaux libres ainsi que la libération massive de médiateurs de l'inflammation va conduire à une destruction du tissu conjonctif de la gencive. Les pathologies qui apparaissent sont les gingivites et les parodontites. Il est connu que les abrasifs actuels pour prévenir l'apparition de la plaque dentaire sont généralement trop puissants. Ils ont ainsi comme inconvénients d'irriter la gencive et d'abîmer l'émail des dents.

Les états inflammatoires de la gencive, sont courants. En plus d'être causés par la présence de plaque dentaire, ils peuvent également résulter d'un brossage des dents trop agressif, du port d'appareils dentaires, de tabagisme...Pour répondre à ces inconvénients, il est connu d'utiliser des agents antibactériens. Ces agents antibactériens sont peu sélectifs des bactéries qu'ils éliminent, comme la chlorhexidine par exemple : ils éliminent à la fois les bactéries pathogènes et les non-pathogènes. De plus, ces agents antibactériens ne sont pas recommandés pour une longue utilisation.

Il existe des préparations orales et de soins dentaires contenant des algues comme par exemple le document EP 1 328 285. Ce document présente un produit d'algues qui empêche la formation bactérienne de la plaque dentaire.

En revanche, ce document ne présente pas l'utilisation d'un produit d'algues dans le but de limiter l'inflammation bucco-dentaire, de limiter l'oxydation bucco dentaire et ne traite pas des dégénérescences tissulaires liées aux parodontites.

Ainsi de manière générale, les produits actuels qu'ils soient à base d'algues ou autres, s'adressent principalement à l'activité antibactérienne et peu d'entre eux traitent des états inflammatoires ou des dégénérescences tissulaires liées aux parodontites.

Un autre état de la technique est illustré par le document FR2 914 190 qui utilise un extrait d'algues destiné au traitement de processus inflammatoires. L'enseignement de ce document montre comment obtenir un extrait d'algues ayant une forte teneur en composés phénoliques et son utilisation dans le domaine pharmaceutique destinée au traitement de processus inflammatoire.

Toutefois, l'enseignement de ce document ne présente pas l'intérêt d'associer un actif antibactérien avec un extrait d'algues qui a pour effet d'augmenter la réduction de la plaque dentaire.

Un autre état de la technique est illustré par le document WO2006/027248 qui décrit des plantes de la famille des fucales qui inhibent une enzyme produite par les bactéries dans l'hygiène dentaire. L'inconvénient est que ces plantes agissent seulement sur l'enzyme et non sur les bactéries. Par conséquent, les bactéries sont encore présentes. Un autre inconvénient est que cette solution agit seulement sur le biofilm.

Le but de la présente invention est d'obtenir un produit à base de mélange d'algues qui permet des synergies prophylactiques de l'hygiène bucco-dentaire.

L'invention propose une composition à usage bucco-dentaire permettant notamment de limiter les risques de parodontopathies, cette composition contenant un extrait d'algues brunes et au moins une zéolithe chargée à l'argent comprenant un mélange d'algues avec au moins de l'Ascophyllum nodosum et du Fucus spp. caractérisée en ce que :
- ledit mélange d'algues présente une proportion d'Ascophyllum nodosum d'au moins 90% du poids du mélange et une proportion de Fucus spp. d'au plus 10% du poids du mélange,
- l'extrait est associé à un tensioactif naturel.

Selon des caractéristiques avantageuses de la présente invention :
- le mélange d'Ascophyllum nodosum et de Fucus spp. est inférieur ou égal à 1,5%, de préférence pris dans la plage de valeur entre 0,1 et 1% du poids de la composition, borne comprise,
- le tensioactif naturel est présent inférieur ou égal à 2% du poids de la composition, de préférence de 0,1 à 1,5% ; avantageusement de 0,25 à 1% voire même de 0,5 à 0,7%,
- le tensioactif naturel est un sophorose lipide,
- la zéolithe chargée à l'argent ou le mélange de zéolithes dont au moins une est chargée à l'argent est entre 0,1 et 1% du poids de la composition, de préférence de 0,25 à 0,75%; avantageusement de 0,35 à 0,65%, voire même de 0,5%,
- la ou au moins une zéolithe est un mélange d'oxyde de silicium et d'oxyde d'aluminium,
- La composition est associée à tout excipient nécessaire à sa mise en forme galénique.

D'autre part, la présente invention concerne une utilisation d'une telle composition pour :
- la prévention et/ou le soin dentaire,
- lutter contre le stress oxydatif des cellules de gencives, ou
- lutter contre la formation du biofilm bactérien.

Pour réaliser au moins une de ces utilisations, la composition à base d'algues et d'au moins une zéolithe chargée à l'argent est intégrée dans un dentifrice ou dans une solution pour bain de bouche ou dans un outil pour un usage dentaire ou buccal.

Les avantages de la présente invention sont entre autres, d'entretenir correctement les gencives et les dents en remédiant aux inconvénients de l'art antérieur. La présente invention propose une solution pour un usage quotidien dans la prévention et le traitement de l'installation de la plaque dentaire.

La présente invention est basée sur la découverte inattendue qu'une composition à base d'extrait d'algues brunes et d'au moins une zéolithe chargée à l'argent comprenant un mélange d'algues avec au moins de l'Ascophyllum nodosum et du Fucus spp. présente des effets importants sur la prévention et le traitement buccodentaire.

Par Fucus spp., il est entendu par toutes les sous espèces de Fucus.

La composition à base d'extrait d'algues et d'au moins une zéolithe chargée à l'argent comprend un tensioactif naturel permettant notamment de lutter efficacement contre le stress oxydatif des cellules de gencives et la formation du biofilm bactérien.

De manière inattendue et avec un effet de symbiose, la présente invention permet à partir de l'association d'un actif antibactérien sous forme de zéolithe ou d'un mélange de zéolithes chargés à l'argent avec un mélange d'algues brunes de favoriser efficacement la lutte contre la formation du biofilm bactérien.

On comprendra mieux l'invention à l'aide de la description faite ci-après d'un mode de mise en oeuvre donné à titre d'exemple non limitatif, en référence aux figures annexées suivantes :
- la figure de référence 1 est une représentation de l'effet anti-inflammatoire à l'aide de fibroblastes de gencives pour la réduction d'interleukine 6.
- la figure de référence 2 est une représentation de l'effet anti-inflammatoire à l'aide de fibroblastes de gencives pour la réduction du facteur α de nécrose tumorale.
- la figure de référence 3 est une représentation de l'effet anti-inflammatoire sur des macrophages pour l'inhibition de la production d'interleukine 6.
- la figure de référence 4 est une représentation de l'effet anti-inflammatoire sur des macrophages pour l'inhibition de la production du facteur a de nécrose tumorale.
- la figure de référence 5 est une représentation de l'effet antioxydant sur des cellules de l'épithélium.

L'objet de la présente invention est l'utilisation d'une composition à base d'extrait d'algues et d'au moins une zéolithe chargée à l'argent destinée à l'usage hygiène bucco-dentaire, cet usage pouvant concerner la prévention, le soin ou l'hygiène.

La présente invention a pour but de prévenir des problèmes bucco-dentaires en luttant contre les trois effets mentionnés ci-après liés notamment à la formation de la plaque dentaire.

Selon la présente invention, la composition à usage bucco dentaire d'extrait d'algues brunes et d'au moins une zéolithe chargée à l'argent comprenant un mélange d'algues avec au moins de l'Ascophyllum nodosum et du Fucus spp. est caractérisée en ce que la proportion d'Ascophyllum nodosum est d'au moins 90% et que la proportion de Fucus spp. est d'au plus 10%, et en ce que ledit extrait est associé à un tensioactif naturel.

L'Ascophyllum nodosum et les espèces de Fucus, notamment mais non limitativement le Fucus vesiculosus, sont des types d'algues brunes répandues.

On sait qu'un tensioactif modifie la tension superficielle entre deux surfaces en présentant deux parties de polarité différente, l'une lipophile et apolaire et l'autre hydrophile et polaire. Il existe de nombreux tensioactifs d'origine naturelle. On pourra citer entre autres les tensioactifs à base de sucre ou glucosides ou les tensioactifs du type acylglutamates.

Comme tensioactif d'origine naturelle, selon l'invention, il est préféré un sophorose lipide. Le sophorose est un diholoside existant dans la nature. Il est constitué de deux unités de glucose reliées par une liaison osidique. C'est un isomère du maltose et du saccharose. Le sophorose peut être associé avec des lipides pour donner des sophoroses lipides ou sophorolipides qui ont la particularité de réduire la tension de surface de l'eau.

De manière générale, une zéolithe est un minéral microporeux appartenant au groupe des silicates, sous groupe des tectosilicates dans lequel ils forment une famille comprenant des aluminosilicates hydratés de métaux des groupes IA et IIA du tableau périodique des éléments, tels le calcium, le magnésium et le potassium.

Il existe deux sortes de zéolithe, la naturelle et la synthétique. Ces zéolithes sont des polymères inorganiques cristallins structurellement complexes, basés sur une suite indéfinie tridimensionnelle de structures quadri-connectées d'oxyde d'aluminium, notamment du AlO_{4,} et d'oxyde de silicium, notamment du SIO₄ tétraédriques, ces structures étant liées entre elles par un échange d'ion oxygène. Chaque AlO₄ tétraédrique présent dans la structure apporte une forte charge négative qui est contre balancée par un ou plusieurs cations, tels que Ca²⁺, Mg²⁺ ou K⁺.

Il est entendu que la zéolithe incluse dans la composition ou au moins une zéolithe du mélange de zéolithes permet la libération lente d'ions d'argent, qui possèdent un effet anti-microbien, c'est-à-dire une famille de substances qui tue les bactéries ou ralentit la croissance des microbes. En complément, il est aussi possible d'utiliser des ions de cuivre et/ou de zinc.

Les ions de la zéolithe ou du mélange de zéolithes sont activés sur demande en présence d'humidité ou d'un agent activant. Par exemple, les ions argent combattent les micro-organismes de trois façons : en privant lesdits micro-organismes de nourriture, en les stérilisant et en les étouffant.

Un des effets pour la lutte contre la formation du biofilm bactérien est l'effet anti-inflammatoire, un autre effet est l'antioxydant et le dernier effet est l'antibactérien. Ceci va être expliqué dans les trois exemples suivants.

### Exemple de référence 1: effet anti-inflammatoire

Dans cet exemple, il est démontré l'effet anti-inflammatoire en prenant des fibroblastes de gencives qui sont incubés pendant 2h avec l'extrait d'algues sélectionné.

On obtient un extrait polyphénolique d'algues brunes par un procédé de fabrication qui comprend les étapes suivantes : broyage des algues fraiches, extraction aqueuse, séparation solide/liquide, filtration pour l'élimination de micro-particules, élimination des alginates par précipitation, élimination de l'iode, puis séchage par atomisation.

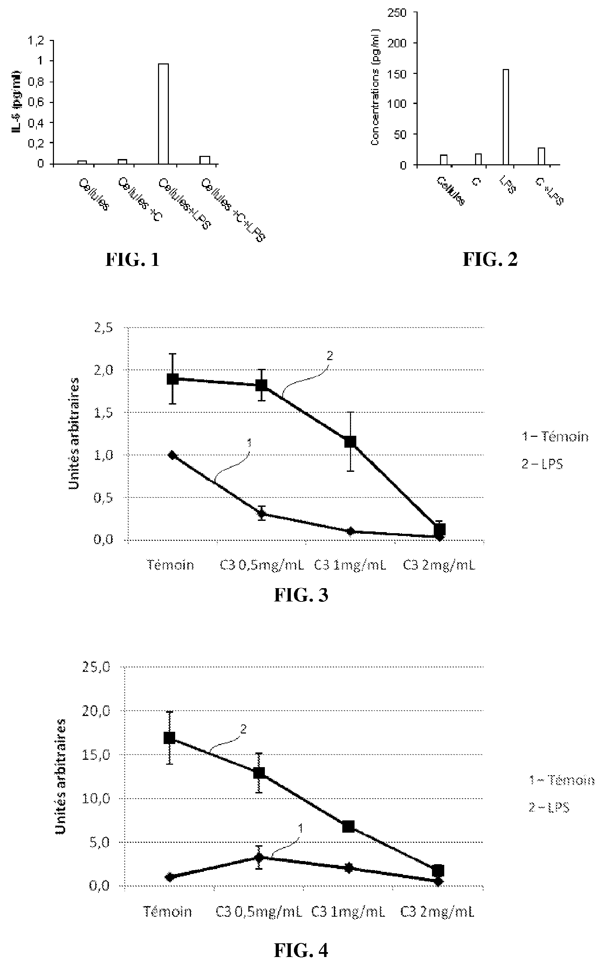

Cet extrait est un extrait d'algues brunes qui est un mélange de 90% d'Ascophyllum nodosum et de 10% de Fucus spp. Ces fibroblastes sont ensuite soumis à un stress inflammatoire à l'aide de l'agent biologique lipopolysaccharide d'Escherichia Coli pendant 24h. Les résultats des différents médiateurs de l'inflammation sont dosés et sont illustrés aux figures 1 et 2.

Sur ces deux figures, l'extrait d'algues sans zéolithe est noté C, les cellules correspondent à des fibroblastes de gencives et un agent biologique lipopolysaccharide de la souche d'Escherichia Coli est noté LPS.

La figure de référence 1 est une représentation de l'effet anti-inflammatoire à l'aide de fibroblastes de gencives pour la réduction d'interleukine 6. Il est entendu par interleukine 6 une cytokine clé dans la régulation de l'inflammation aigüe et chronique. Il est connu qu'une hyperproduction d'interleukine 6 peut provoquer une inflammation.

La figure de référence 1 permet d'apprécier tout d'abord que l'extrait C n'induit pas de stress inflammatoire car les fibroblastes de gencives ne réagissent pas avec l'extrait d'algues. Ensuite, elle permet de voir que l'effet anti-inflammatoire de l'extrait C est vérifié. En effet, en le comparant avec un premier mélange de cellules et de LPS avec un deuxième mélange de cellules, de LPS et de l'extrait d'algues, il est observé que l'extrait d'algues limite l'impact du LPS. Par conséquent, les résultats indiquent que l'extrait d'algues permet de réduire la production d'interleukine 6.

La figure de référence 2 est une représentation de l'effet anti-inflammatoire à l'aide de fibroblastes de gencives pour la réduction de la production du facteur α de nécrose tumorale. Il est entendu par facteur α de nécrose tumorale un révélateur de signe de l'inflammation en mesurant sa concentration. En effet, une augmentation locale de la concentration de facteur α de nécrose tumorale est significative des signes cardinaux d"inflammation comme rougeur, chaleur, tuméfaction, douleur...

De la même manière que la figure 1, la figure 2 montre que l'extrait C n'induit pas de stress inflammatoire car les fibroblastes de gencives ne réagissent pas avec l'extrait d'algues. Elle confirme que l'effet anti-inflammatoire de l'extrait C est vérifié, en comparant un premier mélange comprenant des cellules et du LPS avec un deuxième mélange comprenant des cellules, du LPS et de l'extrait d'algues, il est observé que l'extrait d'algues limite l'impact du LPS. Par conséquent, les résultats indiquent que l'extrait d'algues permet de réduire la production du facteur α de nécrose tumorale.

Afin de caractériser la dose d'extrait d'algue responsable de l'effet anti-inflammatoire, il a été réalisé un protocole expérimental sur des macrophages qui sont des cellules majeures de la réponse inflammatoire.

Sur les figures de référence 3 et 4 l'extrait d'algues est noté C3 et correspond à un mélange d'extrait d'algues d'Ascophyllum nodosum et de Fucus spp. Ces figures représentent le résultat du protocole expérimental. Ainsi, des macrophages ont été incubés dans un premier temps avec différentes concentrations de l'extrait d'algues pendant 2 heures avant d'être stimulés par un stress inflammatoire avec LPS à 0,5 µg/mL pendant 24 heures. Les cytokines proinflammatoires interleukine 6 et le facteur α de nécrose tumorale sont dosés dans le surnageant cellulaire par une technique biochimique du type ELISA traduit par dosage d'immunoadsorption par enzyme liée.

Les résultats de la figure de référence 3 montrent que l'extrait d'algues inhibe l'inflammation induite par la production d'interleukine 6 par les macrophages stimulés avec l'agent biologique lipopolysaccharide, de manière dépendante de la dose. En effet, plus la quantité de C3 augmente et plus la production d'interleukine 6 diminue, ce qui montre bien la dépendance avec la dose administrée.

De la même manière, la figure de référence 4 montre le même résultat concernant la production du facteur α de nécrose tumorale.

Ces deux figures montrent que l'extrait d'algues est un agent de limitation efficace de l'inflammation.

### Exemple de référence 2: effet antioxydant

Du fait de sa structure chimique, l'extrait d'algues possède un potentiel antioxydant intrinsèque.

Une première série d'essais a été réalisée in vitro, sur des modèles acellulaires :
- Evaluation de la mesure des capacités antioxydantes dans les échantillons biologiques : l'extrait marin a une activité de 296 µmol Eq Trolox/g
- Evaluation de la capacité de piégeage de l'anion superoxyde : l'extrait d'algue à 0.1% a une activité de 87 SOD/min.
- Evaluation de la capacité de piégeage du radical hydroxyle : l'extrait d'algue à 1% a une activité de 1556 Eq Acide benzoïque (mg/l).

Ces trois séries d'essais indiquent que l'extrait d'algues sans zéolithe a la capacité d'inhiber la formation de radicaux libres, c'est-à-dire ayant une capacité antioxydante, mais également de piéger des espèces réactives de l'oxygène, c'est-à-dire un anion superoxyde et un radical hydroxyle. L'extrait d'algues agit donc en prévention d'un stress oxydant mais également en réparation des composés oxydatifs déjà présents.

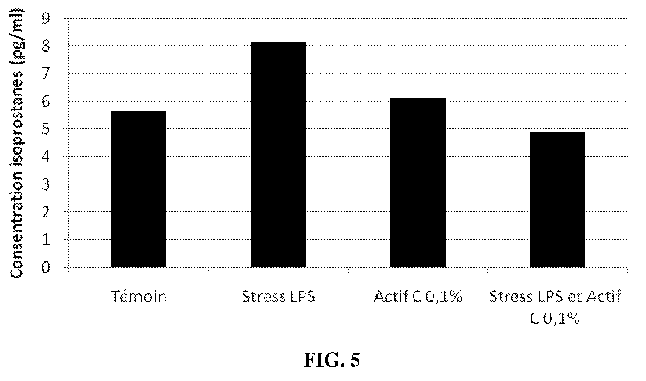

Afin de confirmer la réalité physiologique de ces résultats, les effets antioxydants de cet extrait à l'échelle cellulaire ont été étudiés. Dans un premier temps des cellules de l'épithélium gingival ont été incubées pendant 1 heure avec l'extrait d'algues à 0,1% noté actif C sur la figure 5. Ensuite, le stress oxydant est stimulé avec l'agent biologique lipopolysaccharide noté LPS sur la figure de référence 5 de la souche Porphyromonas Gingivalis à 10 µg/mL pendant 2 heures. Les isoprostanes sont formés par un processus radicalaire non enzymatique de peroxydation des acides gras polyinsaturés, c'est-à-dire des acides arachidoniques des phospholipides membranaires. La mesure de la production d'isoprostanes dans le surnageant cellulaire se fait par LCMS c'est-à-dire la chromatographie phase en liquide/spectrométrie de masse.

La figure de référence 5 illustre les résultats de l'effet antioxydant. En effet, les résultats de la figure de référence 5 montrent que l'extrait d'algues inhibe le stress oxydant induit sur les cellules gingivales par l'agent biologique lipopolysaccharide. Il peut être apprécié que la quantité d'isoprostanes en pg/ml induite par le mélange LPS et actif C est plus petite que la quantité d'isoprostanes induite par le stress LPS.

D'autre part, l'extrait d'algues est appliqué avant le stress oxydant de sorte à prévenir les dommages induits par le stress oxydant. Les données de la figure de référence 5 montrent bien que le mélange du stress de l'agent biologique lipopolysaccharide et de l'extrait d'algues noté C agit en réduisant les dommages d'un stress oxydant. En effet, nous pouvons apprécier que la quantité d'isoprostanes en pg/ml induite par le mélange LPS et actif C est plus petite que la quantité d'isoprostanes induite par l'actif C.

L'exemple 3 suivant montre la validation de l'effet antibactérien.

### Exemple 3 : effet antibactérien

Les effets antibactériens sont testés sur les souches de la flore buccale impliquée dans les parodontites. Préférentiellement il a été choisi un Porphyromonas gingivalis ainsi qu'une bactérie commensale, comme par exemple le Streptococcus gordonii, c'est-à-dire des bactéries qui vivent des déchets qui se trouvent à l'extérieur des tissus. La présente invention montre ainsi son intérêt par l'association de l'extrait d'algues à d'autres ingrédients connus pour leur rôle antibactérien afin de prévenir la formation du biofilm bactérien.

Voici un tableau qui représente l'association d'une zéolithe d'argent et de l'extrait d'algues :

| | | Zéolithe d'argent | Extrait d'algue | Zéolithe d'argent +extrait d'algue |
|---|---|---|---|---|
| Activité Antibactérienne Exprimée en Unité formant une Colonie/mL | P. gingivalis | ↓1,60x10⁷ ±0,05 | Pas d'effet | ↓2,46x10⁷ ±0,16 |
| | S. gordonii | Pas d'effet | Pas d'effet | Pas d'effet |
| Activité antibiofilm (effet préventif) Exprimée en % d'infection | P. gingivalis | 88,81 ±0,22 | Pas d'effet | <0 |
| | S. gordonii | 49,66 ±0,34 | Pas d'effet | <0 |
| | P. gingivalis + S. gordonii | 93,66 ±0,13 | Pas d'effet | 73,26 ±2,09 |

L'analyse de ce tableau permet de faire ressortir que le mélange de la 0.5% zéolithe et 0.5% de l'extrait d'algues a l'avantage de ne pas s'accompagner d'effets sur la flore commensale de la bouche. En effet, comme les résultats du tableau l'indiquent, il n'y a pas d'effet avec le Streptococcus gordonii qui simule un ensemble complexe de bactéries et de protozoaires, cette flore étant indispensable au bon équilibre bactérien bucco-dentaire.

D'autre part, le mélange de 0.5% zéolithe et de 0.5% d'extrait d'algues montre une augmentation inattendue de l'effet anti bactérien.

D'une autre manière que la zéolithe, l'association avec un tensioactif ayant un effet antibiofilm sur Porphyromonas gingivalis et Streptococcus gordonii peut être employé.

C'est pourquoi, la combinaison de 0.5% de zéolithe ou d'un mélange de zéolithes, comprenant de l'argent, de 0.5% d'un actif marin à base d'extrait d'algues et d'un tensioactif permet d'obtenir un produit avec un fort pouvoir antibactérien et un effet anti-biofilm puissant. Ces deux éléments sont importants à une bonne hygiène bucco-dentaire.

Ainsi la composition selon la présente invention peut avoir plusieurs utilisations qui sont non limitatives:
- une utilisation pour la prévention et/ou le soin dentaire,
- une utilisation pour lutter contre le stress oxydatif des cellules de gencives.
- une utilisation pour lutter contre la formation du biofilm bactérien.

La composition peut également être associée à tout excipient nécessaire à sa mise en forme galénique telle qu'un gélifiant, un aromatisant, un agent abrasif, un colorant, un opacifiant...

Afin de permettre ces utilisations, la composition peut être intégrée dans un dentifrice, dans une solution pour bains de bouche ou dans un outil de traitement des dents ou de la bouche. Comme exemples d'outils, il peut être mentionné non limitativement une brosse à dent ou du fil dentaire auxquels cas la composition est intégrée dans les poils de la brosse ou sur le fil, par exemple en tant que revêtement de ceux-ci.

## Revendications

1. Composition à usage bucco-dentaire d'extrait d'algues brunes comprenant un mélange d'algues avec au moins de l'Ascophyllum nodosum et du Fucus spp. étant **caractérisée en ce que** ledit mélange d'algues présente une proportion d'Ascophyllum nodosum d'au moins 90% du poids du mélange et que la proportion de Fucus spp. est d'au plus 10% du poids du mélange, et **en ce que** ledit extrait est associé à au moins une zéolithe chargée à l'argent.

2. Composition selon la revendication 1, **caractérisée en ce que** le mélange d'Ascophyllum nodosum et de Fucus spp. est inférieur ou égal à 1,5% du poids de la composition de préférence de 0,1 et 1%.

3. Composition selon les revendications 1 et 2, **caractérisée en ce qu'**un tensioactif naturel est présent inférieur ou égal à 2% du poids de la composition.

4. Composition selon la revendication 3, **caractérisée en ce que** le tensioactif naturel est un sophorose lipide.

5. Composition selon la revendication 1, **caractérisée en ce que** la zéolithe ou le mélange de zéolithes est entre 0,1 et 1% du poids de la composition.

6. Composition selon la revendication 1, **caractérisée en ce que** la zéolithe ou au moins une zéolithe est un mélange d'oxyde de silicium et d'oxyde d'aluminium.

7. Composition selon l'une quelconque des revendications 1 à 6, **caractérisée en ce qu'**elle est associée à tout excipient nécessaire à sa mise en forme galénique.

8. Composition selon l'une quelconque des revendications 1 à 7 pour son utilisation dans le traitement antibactérien.

9. Composition selon l'une quelconque des revendications 1 à 7 pour son utilisation dans le traitement des maladies liées à la formation du biofilm bactérien défavorable pour la gencive.

10. Dentifrice ou solution pour bain de bouche ou outil pour un usage dentaire ou buccal comprenant la composition selon l'une quelconque des revendications 1 à 7.

## Patentansprüche

1. Zusammensetzung eines Braunalgenextrakts zur Verwendung im Mund-Zahnbereich, umfassend eine Algenmischung mit mindestens Ascophyllum nodosum und Fucus spp., **dadurch gekennzeichnet, dass** die Algenmischung einen Anteil von Ascophyllum nodosum von mindestens 90% des Gewichts der Mischung aufweist und dass der Anteil an Fucus spp. maximal 10% des Gewichts der Mischung beträgt, und dadurch, dass der Extrakt mit mindestens einem mit Silber beladenen Zeolithen kombiniert ist.

2. Zusammensetzung nach Anspruch 1, **dadurch gekennzeichnet, dass** die Mischung aus Ascophyllum nodosum und Fucus spp. weniger als oder gleich 1,5% des Gewichts der Zusammensetzung, vorzugsweise 0,1 und 1%, ist.

3. Zusammensetzung nach den Ansprüchen 1 und 2, **dadurch gekennzeichnet, dass** ein natürliches Tensid in weniger als oder gleich 2% des Gewichts der Zusammensetzung vorliegt.

4. Zusammensetzung nach Anspruch 3, **dadurch gekennzeichnet, dass** das natürliche Tensid ein Sophoroselipid ist.

5. Zusammensetzung nach Anspruch 1, **dadurch gekennzeichnet, dass** der Zeolith oder die Zeolithmischung zwischen 0,1 und 1% des Gewichts der Zusammensetzung ist.

6. Zusammensetzung nach Anspruch 1, **dadurch gekennzeichnet, dass** der Zeolith oder mindestens ein Zeolith eine Mischung aus Siliziumoxid und Aluminiumoxid ist.

7. Zusammensetzung nach einem der Ansprüche 1 bis 6, **dadurch gekennzeichnet, dass** sie mit jeglichem Grundstoff, der für ihre galenische Formgebung erforderlich ist, kombiniert ist.

8. Zusammensetzung nach einem der Ansprüche 1 bis 7 zur Verwendung in der antibakteriellen Behandlung.

9. Zusammensetzung nach einem der Ansprüche 1 bis 7 zur Verwendung in der Behandlung von Krankheiten, die mit der Bildung eines dem Zahnfleisch abträglichen, bakteriellen Biofilms assoziiert sind.

10. Zahnpaste oder Mundspülungslösung oder Gerät zur Verwendung im Zahn- oder Mundbereich, umfassend die Zusammensetzung nach einem der Ansprüche 1 bis 7.

## Claims

1. Composition for buccodental use of extract of brown algae comprising a mixture of algae with at least *Ascophyllum nodosum* and *Fucus* spp., which is **characterized in that** said mixture of algae has a proportion of *Ascophyllum nodosum* of at least 90% of the weight of the mixture and the proportion of *Fucus* spp. is at most 10% of the weight of the mixture, and **in that** said extract is combined with at least one silver-loaded zeolite.

2. Composition according to Claim 1, **characterized in that** the mixture of *Ascophyllum nodosum* and *Fucus* spp. is less than or equal to 1.5% of the weight of the composition, preferably from 0.1% to 1%.

3. Composition according to Claims 1 and 2, **characterized in that** a natural surfactant is present at less than or equal to 2% of the weight of the composition.

4. Composition according to Claim 3, **characterized in that** the natural surfactant is a sophorolipid.

5. Composition according to Claim 1, **characterized in that** the zeolite or the mixture of zeolites is between 0.1% and 1% of the weight of the composition.

6. Composition according to Claim 1, **characterized in that** the zeolite or at least one zeolite is a mixture of silicon oxide and aluminium oxide.

7. Composition according to any one of Claims 1 to 6, **characterized in that** it is combined with any excipient required for galenical forming thereof.

8. Composition according to any one of Claims 1 to 7, for use thereof in antibacterial treatment.

9. Composition according to any one of Claims 1 to 7, for use thereof in the treatment of diseases associated with the formation of the bacterial biofilm unfavourable to the gums.

10. Toothpaste or solution for a mouthwash or tool for dental or buccal use, comprising the composition according to any one of Claims 1 to 7.
